# EUROPEAN PATENT SPECIFICATION

(11) **EP 3 253 304 B1**
(45) Date of publication and mention of the grant of the patent: **08.07.2020**
(21) Application number: 16716110.8
(22) Date of filing: 28.03.2016
(51) Int. Cl.: A61B 17/12

(54) **EMBOLIC COIL DELIVERY SYSTEM WITH EASY-RELEASE KNOT**
SYSTEM ZUR EINFÜHRUNG EINER EMBOLIESPIRALE MIT LEICHT LÖSBAREM KNOTEN
SYSTÈME DE POSE DE SPIRE MÉTALLIQUE D'EMBOLISATION AVEC NOEUD À LIBÉRATION FACILE

(30) Priority: 26.03.2015 US 201562138498 P
(43) Date of publication of application: 13.12.2017
(73) Proprietor: Boston Scientific Scimed, Inc., Maple Grove, MN 55311-1566 (US)
(72) Inventor: ANDERSON, Mary-Claire, St. Louis Park, MN 55416 (US); TASSONI, Nicholas, Ramsey, MN 55303 (US); GUPTA, Ajay, Shoreview, MN 55126-6153 (US)
(74) Representative: Vossius & Partner Patentanwälte Rechtsanwälte mbB
(86) International application number: PCT/US2016/024581
(87) International publication number: WO 2016/154632

(56) References cited:
- WO-A2-2012/092351
- US-A1- 2005 085 843

## Description

### FIELD OF THE INVENTION

The present disclosure relates to the field of medical devices. Specifically, the present disclosure relates to systems for releasing an embolic coil from a delivery device into the vasculature of a patient. More specifically, the present disclosure relates to a detachment mechanism that allows controlled deployment of an embolic coil beyond the distal opening of the delivery device without embolic coil kickback.

### BACKGROUND

Various medical conditions require partial or complete occlusion of blood vessels or vascular malformations (e.g., an aneurysm). Embolic coils have proven popular for such applications owing to their ability to be placed at such sites using a variety of percutaneous delivery techniques. To achieve adequate embolic coil density for embolus formation, it is common for multiple embolic coils to be implanted within the vascular malformation. Embolic coils are typically delivered to a selected site within the vasculature using a catheter-based delivery system in a minimally invasive procedure. A common delivery system design includes an embolic coil secured by a tether in the form of a simple loop that extends the length of the delivery system, passes through an attachment member on the embolic coil and extends back along the length of the delivery system. The embolic coil is reversibly retained against the distal end of the delivery system by applying tension to both free ends of the tether. The embolic coil is deployed by releasing one end of the tether while retracting the other of the tether until the free end is completely removed from the attachment member. A common problem associated with this simple loop design is that pulling the free end of the tether through the attachment member tends to cause a portion of the recently released embolic coil to protrude out of the occlusion site into the parent vessel. This partial displacement of the embolic coil, often referred to as kickback, can disrupt the flow of blood in the parent vessel and potentially lead to thrombosis in an undesired location. Document WO2012/092351 discloses an embolic coil delivery system according to the preamble of claim 1.

Accordingly, there is a continued need for a 100% detachable delivery system that allows the embolic coil to be advanced completely outside the distal end of the delivery system without deploying, and provides complete release of the embolic coil at the desired time.

### SUMMARY

The present invention is defined in the appended set of claims. Particular embodiments of the disclosure are described in the Summary and Detailed Description of the Preferred Embodiments, below. Although the disclosure has been described in connection with specific embodiments, it should be understood that the disclosure as claimed should not be unduly limited to such specific embodiments. For example, the systems described herein provide controlled embolic coil deployment beyond the distal opening of the microcatheter without embolic coil kickback.

In one aspect, the present disclosure relates to an embolic coil delivery system, comprising: a microcatheter that includes a proximal end defining a proximal aperture, a distal end defining a distal aperture and a lumen extending therebetween; a delivery tube slidably disposed within the lumen of the microcatheter, wherein the delivery tube includes a proximal end defining a proximal aperture, a distal end defining a distal aperture and a lumen extending therebetween; an embolic coil that includes a proximal attachment member configured to reversibly engage the distal end of the delivery tube; and a tether comprising a first end and a second end, wherein the tether forms a loop along the length of the delivery tube lumen, and wherein a portion of the tether forms a releasable knot around the proximal attachment member of the embolic coil. The first and second ends of the tether may extend outside the proximal end of the microcatheter. The lumen of the microcatheter may define an inner diameter of about 0.021 inches (0.533 mm) to about 0.027 inches (0.690 mm). The delivery tube may include an outer diameter of at most about 0.019 inches (0.483 mm). The lumen of the delivery tube may define an inner diameter of about 0.017 inches (0.432 mm). The attachment member may include an outer diameter that is substantially equal to the outer diameter of the delivery tube. For example, the attachment member may include an outer diameter of about 0.017 inches (0.432 mm). The tether may include an outer diameter of about 0.003 inches (0.080 mm). The releasable knot may be a slip-knot. For example, the releasable knot may be a highwayman's knot. Applying tension to the first end of the tether may tighten the releasable knot around the proximal attachment member of the embolic coil. Applying tension to the first end of the tether may secure the proximal attachment member of the embolic coil to the distal end of the delivery tube. Applying tension to the second end of the tether may release the releasable knot from the proximal attachment member of the embolic coil. A portion of the proximal attachment member may extend into the distal aperture of the delivery tube when the proximal attachment member is secured to the distal end of the delivery tube. The releasable knot does not extend outside the distal end of the delivery tube when the releasable knot is attached to the proximal attachment member of the embolic coil. The attachment member may comprise an eyelet defining an opening. The releasable knot may pass through the opening of the eyelet. At least a portion of the eyelet may be curved. The inner and/or outer surfaces of the eyelet may be substantially smooth. The attachment member may be formed from a proximal winding of the embolic coil. The attachment member may be fixedly attached to the proximal end of the embolic coil by a weld, solder or glue. The tether may be a flexible line, a filament, a monofilament, a multifilament, a braid, a stretch-resistant member, a suture material or a metallic wire. The metallic wire may be formed from nitinol. The first and second ends of the tether may be operatively joined to an actuator. The actuator may comprise a handle and a thumbwheel. The embolic coil may be a fibered embolic coil. The embolic coil may be comprised of a metal such as platinum, rhodium, palladium, rhenium, tungsten, gold, silver, tantalum, and alloys of these metals including platinum/tungsten alloys and nickel-titanium alloys.

In another aspect, which is not part of the invention, the present disclosure relates to a method of delivering an embolic coil, comprising: advancing an embolic coil delivery system to a target occlusion area within a patient; advancing delivery tube through the lumen of the microcatheter such that the distal end of the delivery tube extends outside the distal aperture of the microcatheter; and applying tension to the second end of the tether to release the proximal attachment member from the distal aperture of the delivery tube, thereby releasing the embolic coil into the target occlusion area. The delivery system may comprise a microcatheter that includes a proximal end defining a proximal aperture, a distal end defining a distal aperture and a lumen extending therebetween. A delivery tube may be slidably disposed within the lumen of the microcatheter, wherein the delivery tube includes a proximal end defining a proximal aperture, a distal end defining a distal aperture and a lumen extending therebetween. An embolic coil that includes a proximal attachment member may be configured to reversibly engage the distal end of the delivery tube. A tether comprising a first end and a second end that forms a loop along the length of the delivery tube lumen, may form a releasable knot around the proximal attachment member of the embolic coil. Applying tension to the first end of the tether may secure the proximal attachment member of the embolic coil to the distal aperture of the delivery tube. The method may further include retracting the delivery tube through the lumen of the microcatheter such that the distal end of the delivery tube does not extend outside the distal aperture of the microcatheter. The method may further include removing the delivery system from the patient. The method may still further include, prior to releasing the embolic coil, advancing or retracting the delivery tube to position the embolic coil within the target occlusion area. The method may still further include removing the delivery tube from the lumen of the microcatheter. A second embolic coil may be loaded into the delivery tube. The delivery tube loaded with the second embolic coil may be advanced through the lumen of the microcatheter to a target occlusion area. The second embolic coil may be released the embolic coil into the target occlusion area.

In yet another aspect, the present disclosure relates to a kit for delivery of an embolic coil, comprising: a microcatheter defining a lumen disposed along an axis, the microcatheter having a proximal end defining a proximal aperture, a distal end defining a distal aperture, an inner diameter and an outer diameter; a delivery tube defining a lumen disposed along an axis, the delivery tube having a proximal end defining a proximal aperture, a distal end defining a distal aperture, an inner diameter and an outer diameter, wherein the outer diameter of the delivery tube is less than the inner diameter of the microcatheter; an embolic coil that includes a proximal attachment member configured to reversibly engage the distal aperture of the delivery tube; and a tether comprising a first end and a second end, wherein the tether is disposed along the length of the delivery tube lumen and coupled to the proximal attachment member of the embolic coil by a releasable knot.

Non-limiting embodiments of the present disclosure are described by way of example with reference to the accompanying figures, which are schematic and not intended to be drawn to scale. In the figures, each identical or nearly identical component illustrated is typically represented by a single numeral. For purposes of clarity, not every component is labeled in every figure, nor is every component of each embodiment of the disclosure shown where illustration is not necessary to allow those of ordinary skill in the art to understand the disclosure. In the figures:
FIG. 1 depicts a delivery system in which the delivery tube and attached embolic coil are disposed entirely within the lumen of the microcatheter, in accordance with one embodiment of the present disclosure.
FIG. 2 depicts the delivery system of FIG. 1 after the delivery tube and attached embolic coil have advanced outside the distal end of the microcatheter, in accordance with another embodiment of the present disclosure.
FIG. 3 depicts a magnified view of the delivery tube and attached embolic coil positioned outside the distal end of the microcatheter, in accordance with another embodiment of the present disclosure.
FIGS. 4A-C depict a releasable knot in the tightened (4A), partially released (4B) and completely released (4C) configurations, in accordance with embodiments of the present disclosure.
FIG. 5 depicts the delivery system of FIG. 2 following the release of the embolic coil from the delivery tube, in accordance with yet another embodiment of the present disclosure.
FIG. 6 depicts the delivery system with the delivery tube retracted into the microcatheter following the release of the embolic coil, in accordance with another embodiment of the present disclosure.

It is noted that the drawings are intended to depict only typical or exemplary embodiments of the disclosure. It is further noted that the drawings may not be necessarily to scale. Accordingly, the drawings should not be considered as limiting the scope of the disclosure. The disclosure will now be described in greater detail with reference to the accompanying drawings.

### DETAILED DESCRIPTION OF THE PREFERRED EMBODIMENTS

Before the present disclosure is described in further detail, it is to be understood that the disclosure is not limited to the particular embodiments described, as such may vary. It is also to be understood that the terminology used herein is for the purpose of describing particular embodiments only, and is not intended to be limiting beyond the scope of the appended claims. Unless defined otherwise, all technical terms used herein have the same meaning as commonly understood by one of ordinary skill in the art to which the disclosure belongs. Finally, although embodiments of the present disclosure are described with specific reference to systems and method for delivering embolic coils, it should be appreciated that the scope of the disclosure may be applicable to a number of implantable devices requiring delivery to specific location(s) within a patient, including for example, occlusion balloons, self-expanding stents and the like.

The present disclosure is generally directed to systems and methods for actively releasing medical devices, including, for example, embolic coils within a body lumen of a patient. More specifically, the present disclosure relates to an embolic coil that is 100% detachable and designed to allow the embolic coil to be advanced *(i.e.,* pushed) completely outside the distal end of the delivery microcatheter without deploying. The embolic coil remains attached to the distal end of the delivery tube by an easy-release knot that provides a simple detachment mechanism that is completely controlled by the medical professional. For example, as described in greater detail below, once the delivery tube and attached embolic coil have been advanced outside the distal end of the microcatheter, the medical professional has the option of releasing the embolic coil into the patient or retracting the delivery tube and attached embolic coil back into the microcatheter for repositioning within, or removal from, the patient.

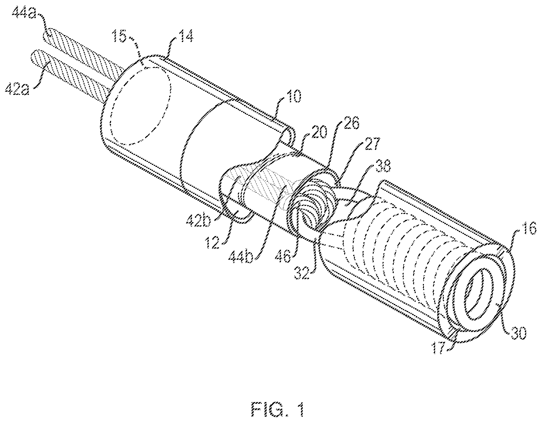

FIG. 1 depicts an embolic coil delivery system, which includes a microcatheter 10 with a proximal end 14 optionally defining a proximal aperture 15, a distal end 16 defining a distal aperture 17 and a lumen 12 extending therebetween. A delivery tube 20 is slidably disposed within the lumen 12 of the microcatheter 10. The delivery tube 20 includes a proximal end (not indicated) optionally defining a proximal aperture (not indicated), a distal end 26 defining a distal aperture 27 and a lumen extending therebetween. An embolic coil 30 is reversibly coupled to the distal end 26 of delivery tube 20 by a proximal attachment member 32, which in preferred embodiments defines a loop or other structure configured to accept a mechanical coupling means as described below. In preferred embodiments, mechanical coupling between the embolic coil 30 and the delivery system is achieved by means of a tether that includes first and second ends 42a, 44a that form a loop along the length of the lumen of delivery tube 20. The first and second ends 42a, 44a of the tether extend outside the proximal end 14 of the microcatheter 10. A portion 42b and 44b of the tether between the first and second ends 42a, 44a is tied to the proximal attachment member 32 by a releasable knot 46. As illustrated in FIG. 1, the proximal attachment member includes an opening 38 through which the releasable knot is secured. The releasable knot 46 is configured such that applying tension to (*i.e.,* pulling) the first end 42a of the tether in a proximal direction relative to the microcatheter tightens the releasable knot about the proximal attachment member 32. In addition to tightening the releasable knot, the tension applied to the first end 42a of the tether reversibly secures the proximal attachment member 32 against distal end 26 of the delivery tube 20.

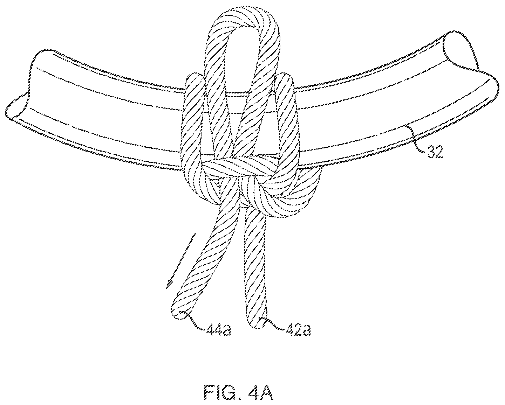

As used herein, the term "tether" refers to a flexible line, including but not limited to, a filament, monofilament, multifilament, braid, suture material or wire (including metallic wire, such as nitinol) capable of being formed into a knot. For example, in one embodiment of the present disclosure, the tether is formed from a suture material or nitinol wire with a diameter of about 0.003 inches (0.080 mm). Similarly, the term "slipknot," "slip-knot" or "easy-release knot," as used herein, refers to a knot formed from a single continuous tether that includes two free ends that extend beyond the proximal end of the microcatheter. Pulling one of the free ends forces the knot to tighten around the structure to which it is attached, while pulling on the other free end will release the knot. While the releasable knot depicted herein (see FIGS. 4A-C) is referred to as a "Highwayman's knot," it should be appreciated that the present disclosure is in no way limited to this specific type of knot, but can include any number of slipknots known in the art, including, for example, a mooring hitch knot, a slipped buntline knot, a tumble hitch knot and the like.

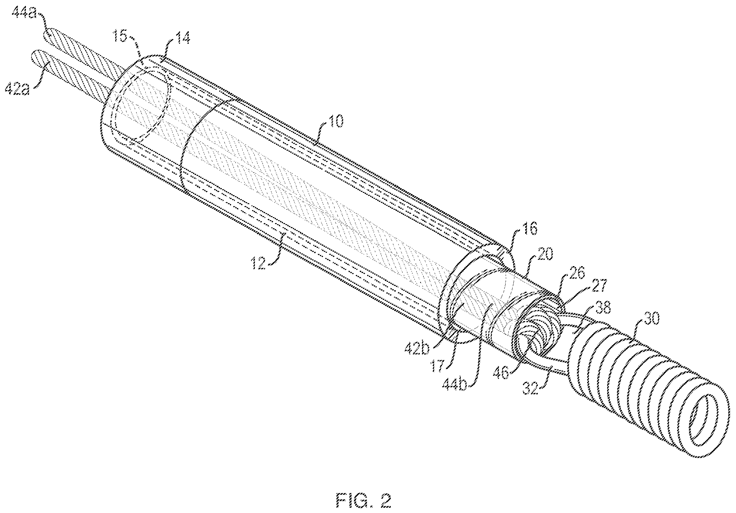

As illustrated in FIG. 2, the delivery tube 20 may be advanced *(i.e.,* pushed) distally through the lumen 12 of the microcatheter 10 until the distal end 26 of delivery tube 20 and embolic coil 30 are disposed outside of distal end 16 of the microcatheter 10. Importantly, the proximal tension on first end 42a of the tether is maintained while the delivery tube is being advanced distally such that the proximal attachment member 32 of embolic coil 30 remains securely coupled to the distal end 26 of delivery tube 20.

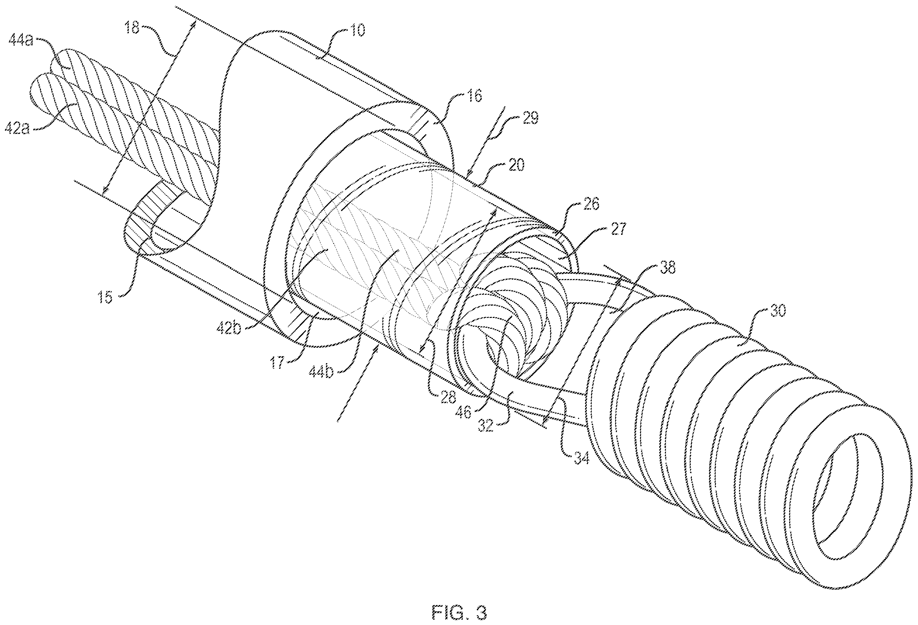

As best illustrated in FIG. 3, the delivery tube 20, proximal attachment member 32 and embolic coil 30 are dimensioned to fit within the lumen 12 of the microcatheter 10. However, the proximal attachment member 32 is dimensioned to only partially fit within the distal aperture of 27 of the delivery tube 20. By way of non-limiting example, in one embodiment the distal aperture 17 of microcatheter 10 has an inner diameter 18 of about 0.021 inches (0.533 mm) to about 0.027 inches (0.690 mm). The delivery tube 20 has a corresponding outer diameter 29 that is slightly smaller, e.g., about 0.019 inches (0.483 mm) than the inner diameter 18 of the microcatheter to permit the delivery tube 20 to slide within the lumen 12 of the microcatheter 10. The distal end 26 of delivery tube 20 further defines a distal aperture 27 with an inner diameter 28 of about 0.017 inches (0.432 mm). Importantly, the proximal attachment member 32 includes a corresponding outer diameter 34 that is approximately equal to the outer diameter 29 of the delivery tube, e.g., about 0.019 inches (0.483 mm).

Providing a proximal attachment member 32 with an outer diameter 34 that is approximately equal to the outer diameter 29 of the delivery tube ensures that the proximal attachment member 32 and embolic coil 30 are able to pass through the lumen 12 of microcatheter 10, but cannot fully enter the lumen of delivery tube 20. This configuration ensures that embolic coil 30 remains reversibly secured to the distal end 26 of the delivery tube 20 until the releasable knot is untied. Of course, all of the dimensions provided above should be viewed only as guidelines, and the disclosure, in its broader aspects, should not be limited thereto.

One advantage associated with retaining the embolic coil 30 outside of the delivery tube 20 is that it allows the thickness of the delivery tube to be increased, thereby imparting greater strength and control (*i.e.,* pushability) for advancing the delivery tube throughout the entire length of the microcatheter.

Another advantage associated with the proximal attachment member 32 described herein is that it includes a portion proximal to the outer diameter 34 that extends partially into the distal aperture 27. This allows the releasable knot 46 to reversibly engage the proximal attachment member entirely within the delivery tube 20. This eliminates safety concerns associated with the releasable knot 46 being exposed to the lining of the vasculature wall and/or microcatheter 10 during deployment of the embolic coil 30. For example, preventing exposure of the releasable knot 46 to the environment outside of the delivery tube limits the likelihood of thrombus formation that could affect the deployment and/or retraction of the embolic coil 30. Preventing exposure of the releasable knot 46 to the microcatheter lumen 12 also limits potential problems with deploying and/or retracting the embolic coil, and minimizes wear *(i.e.,* abrasion) on the tether itself.

Yet another advantage associated with the proximal attachment member 32 described herein is that its curved designs also allows the embolic coil to be re-centered within the distal aperture 27 of the delivery tube 20 if repositioning and/or removal is required (discussed below).

While the proximal attachment member may include a variety of shapes, in one embodiment the proximal attachment member 32 is a curved eyelet, similar to that of a sewing needle. The eyelet may be made of a variety of materials, including the same materials used to form the embolic coil (discussed below). In one embodiment, the eyelet is formed from a proximal winding of the embolic coil. In another embodiment, the eyelet is formed separately from the embolic coil, and attached to the embolic coil by a solder, weld, glue or related bonding techniques known in the art. An eyelet, or similar design, as depicted herein is preferable due to its small size and absence of sharp edges. For example, the smooth surfaces of the eyelet will not cut the tether and/or the vasculature of the patient. Similarly, the small size of the eyelet allows it to remain inside the vascular malformation without extending into the parent vessel.

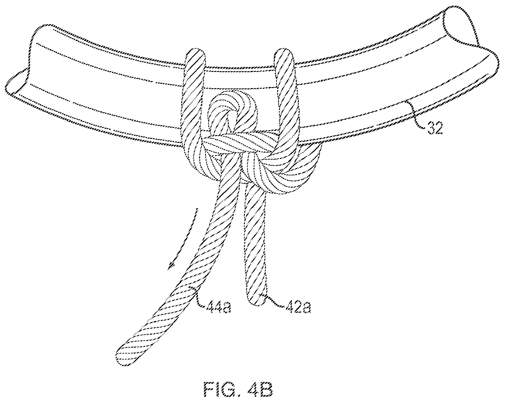

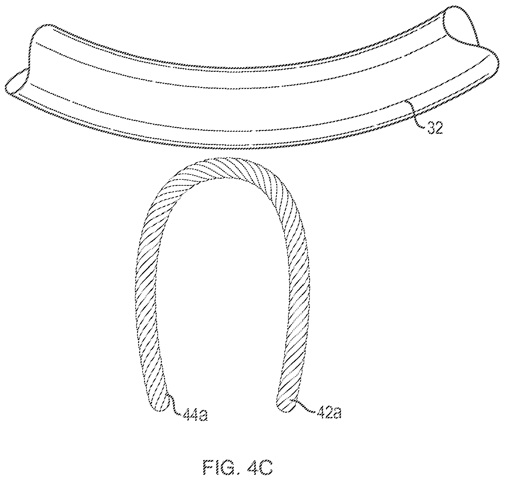

FIG. 4 depicts a magnified view of a releasable knot according to one embodiment of the present disclosure. FIG. 4A specifically depicts a Highwayman's knot secured to a curved portion of the proximal attachment member 32. The knot remains secured to the proximal attachment member by applying continuous tension to the first end 42a of the tether. As discussed above, pulling first end 42a in a proximal direction relative to the microcatheter 10 also ensures that the eyelet remains coupled to the distal end 26 of delivery tube 20. Importantly, little or substantially no tension is applied to second end 44a of the tether when in the tightened configuration. As best depicted in FIG. 4B, the process of releasing the Highwayman's knot from the proximal attachment member 32 begins applying tension to the second end 44a of the tether, while simultaneously relieving at least some of the tension applied to first end 42a. While at least partially relieving some of the tension applied to first end 42a allows the second end 44a of the tether to more easily unwrap from the eyelet, it should be appreciated that the knot may be released while first end 42a remains under tension. As shown in FIG. 4C, continued application of tension to the second end 44a of the tether eventually unties the releasable knot such that no portion of the tether is in contact with the proximal attachment member 32.

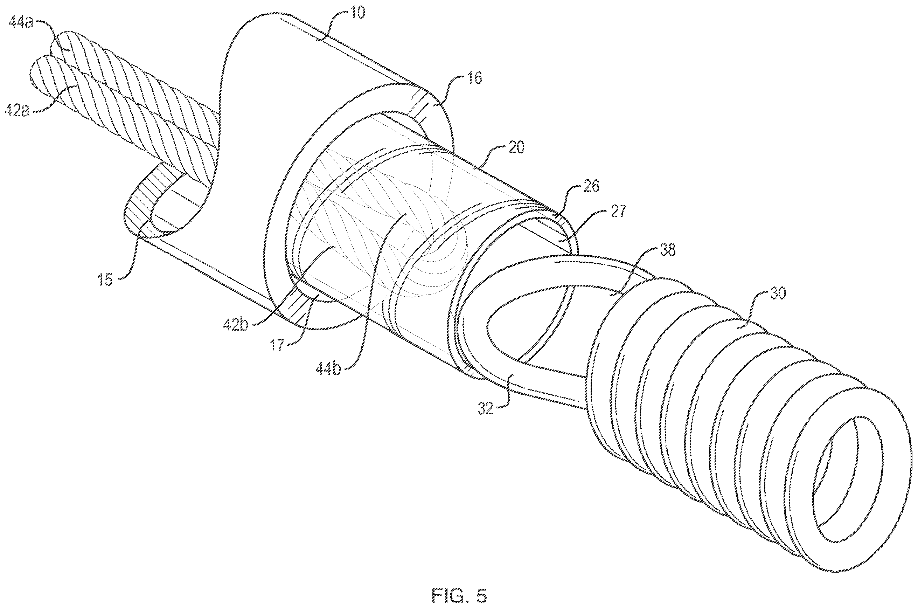

As best depicted in FIG. 5, once the medical professional has verified that the embolic coil 30 is properly positioned within the patient, the embolic coil 30 may be released by untying the knot as discussed in FIGS. 4A-C. In one embodiment, the first and second ends 42a, 44a of the tether extend outside the proximal end 14 of the microcatheter 10 and are secured within an actuator (not shown) that includes a handle and thumbwheel. Such actuators are known in the art, including, for example, U.S. Patent Nos. 8,784,446, 7,285,117, and 6,102,920. The thumbwheel allows the medical professional to control the amount of tension applied to the first or second ends 42a, 44a of the tether as necessary. For example, when the delivery system is being advanced through the vasculature of the patient the thumbwheel may allow the medical professional to maintain the desired amount of tension on the first end 42a of the tether to maintain the proximal attachment member 32 securely coupled to the distal end 26 of the delivery tube 20. Once the embolic coil 30 is properly positioned within the patient, the medical professional can use the thumbwheel to release the tension on the first end 42a of the tether and apply tension on the second end 44a until the knot 46 releases from the eyelet.

Again, the immediate release affected by this knot configuration allows the embolic coil to be 100% detachable since no portion of the tether remains in contact with the proximal attachment member 32 once the knot is untied. The releasable knot 46 is therefore superior to simple loop designs that require the free end of the tether to be pulled all the way through the embolic coil attachment mechanism. As discussed above, the process of removing the tether tends to cause displacement/kickback of the deployed embolic coil into the parent vasculature.

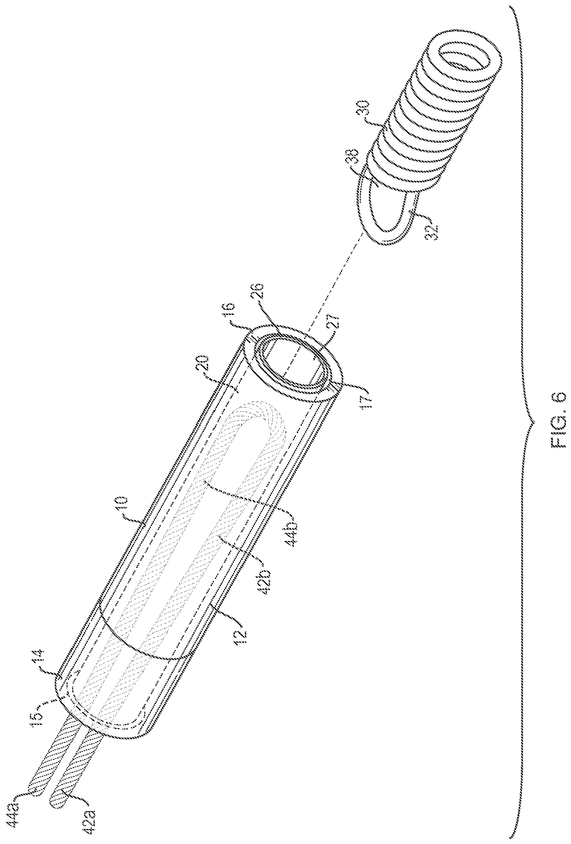

Referring to FIG 6, once the embolic coil is released from the distal end 26 of the delivery tube 20, the delivery tube is retracted into the lumen 12 of the microcatheter 10. The entire delivery system may then be removed from the patient. Alternatively, the delivery tube 20 may be withdrawn from the lumen 12 of the microcatheter 10, and the microcatheter 10 reloaded with another delivery tube 20 containing an embolic coil 30. Once the ends 42a and 44a are secured to the actuator, the delivery tube 20 and attached embolic coil 30 may be advanced through the lumen 12 and distal aperture 17 of the microcatheter 10. Upon verifying that the second embolic coil 30 is properly positioned within the patient, the embolic coil can be released and/or repositioned according the steps described above. This procedure may be repeated as necessary based on the size and shape of the particular vascular malformation.

Referring back to FIGS. 2 and 3, it should be emphasized that if the medical professional determines that the embolic coil 30 is improperly positioned once the delivery tube 20 has been advanced outside of the distal aperture 17 of microcatheter 10, the delivery tube and microcatheter may be retracted into the lumen 12 of the microcatheter for repositioning or removal from the patient.

The embolic coil 30 may be formed from a plurality of coil windings. When manufacturing the embolic coil 30, the coil material is wound into a coil shape, which will typically be linear. Generally speaking, the embolic coil 30 is a metallic coil formed from metals or alloys, for example, selected from platinum group metals, particularly platinum, rhodium, palladium, and rhenium, as well as tungsten, gold, silver, tantalum, and alloys of these metals including platinum/tungsten alloys and nickel-titanium alloys (nitinol) among others. These materials have significant radiopacity, and their alloys may be tailored to have a blend of flexibility and stiffness for the coil. They are also generally biologically inert. The diameter of the wire used in the production of the embolic coil 30 may fall in the range of 0.00025 inches (0.00635 mm) to about 0.006 inches (0.152 mm). The embolic coil 30 may have a primary diameter of between about 0.003 (0.080 mm) and about 0.025 inches (0.635 mm), but for most applications a diameter between about 0.008 inches (0.203 mm) to about 0.018 inches (0.457 mm) provides sufficient hoop strength to hold the embolic coil 30 in place within the chosen body site, lumen or cavity without substantially distending the wall of the site and without moving from the site as a result of the repetitive fluid pulsing found in the vascular system. In one embodiment, the embolic coil is a fibered embolic coil.

All of the devices and/or methods disclosed and claimed herein can be made and executed without undue experimentation in light of the present disclosure. While the devices and methods of this disclosure have been described in terms of preferred embodiments, it will be apparent to those of skill in the art that variations can be applied to the devices and/or methods and in the steps or in the sequence of steps of the method described herein without departing from the scope of the disclosure. All such similar substitutes and modifications apparent to those skilled in the art are deemed to be within the scope of the disclosure as defined by the appended claims.

## Claims

1. An embolic coil delivery system, comprising:
a microcatheter (10) that includes a proximal end (14) defining a proximal aperture (15), a distal end (16) defining a distal aperture (17) and a lumen (12) extending therebetween;
a delivery tube (20) slidably disposed within the lumen of the microcatheter, wherein the delivery tube includes a proximal end defining a proximal aperture, a distal end (26) defining a distal aperture (27) and a lumen extending therebetween;
an embolic coil (30) that includes a proximal attachment member (32) configured to reversibly engage the distal end of the delivery tube, and
a tether comprising a first end (42a) and a second end (44a), wherein the tether forms a loop along a length of the delivery tube lumen, and wherein a portion (42b, 44b) of the tether forms a releasable knot (46) around the proximal attachment member of the embolic coil, wherein a portion of the proximal attachment member extends into the distal aperture of the delivery tube when the proximal attachment member is secured to the distal end of the delivery tube, said embolic coil delivery system being **characterized in that** the proximal attachment member is dimensioned to only partially fit within the distal aperture of the delivery tube.

2. The delivery system of claim 1, wherein the first and second ends of the tether extend outside the proximal end of the microcatheter.

3. The delivery system of claim 1, wherein the releasable knot is a slip-knot or a highwayman's knot.

4. The delivery system of claim 1, wherein applying tension to the first end of the tether tightens the releasable knot around the proximal attachment member of the embolic coil.

5. The delivery system of claim 4, wherein applying tension to the second end of the tether releases the releasable knot from the proximal attachment member of the embolic coil.

6. The delivery system of claim 1, wherein the releasable knot does not extend outside the distal end of the delivery tube when the releasable knot is attached to the proximal attachment member of the embolic coil.

7. The delivery system of claim 1, wherein the attachment member comprises an eyelet defining an opening, the releasable knot passes through the opening of the eyelet, and at least a portion of the eyelet is curved.

## Patentansprüche

1. Emboliespiralen-Abgabesystem, das aufweist:
einen Mikrokatheter (10) mit einem proximalen Ende (14), das eine proximale Öffnung (15) bildet, einem distalen Ende (16), das eine distale Öffnung (17) bildet, und einem sich dazwischen erstreckenden Lumen (12);
eine Abgaberöhre (20), die im Lumen des Mikrokatheters verschiebbar angeordnet ist, wobei die Abgaberöhre aufweist: ein proximales Ende, das eine proximale Öffnung bildet, ein distales Ende (26), das eine distale Öffnung (27) bildet, und ein sich dazwischen erstreckendes Lumen;
eine Emboliespirale (30), die ein proximales Befestigungsteil (32) aufweist, das so konfiguriert ist, dass es einen reversiblen Eingriff mit dem distalen Ende der Abgaberöhre herstellt, und
eine Halteschnur mit einem ersten Ende (42a) und einem zweiten Ende (44a), wobei die Halteschnur eine Schlaufe über die Länge des Abgaberöhrenlumens bildet und wobei ein Abschnitt (42b, 44b) der Halteschnur einen lösbaren Knoten (46) um das proximale Befestigungsteil der Emboliespirale bildet, wobei sich ein Abschnitt des proximalen Befestigungsteils in die distale Öffnung der Abgaberöhre erstreckt, wenn das proximale Befestigungsteil am distalen Ende der Abgaberöhre befestigt ist, wobei das Emboliespiralen-Abgabesystem **dadurch gekennzeichnet ist, dass** das proximale Befestigungsteil so bemessen ist, dass es sich nur teilweise in die distale Öffnung der Abgaberöhre einpasst.

2. Abgabesystem nach Anspruch 1, wobei sich das erste und zweite Ende der Halteschnur außerhalb des proximalen Endes des Mikrokatheters erstrecken.

3. Abgabesystem nach Anspruch 1, wobei der lösbare Knoten ein fortlaufender Knoten oder ein Highwayman's-Hitch-Knoten ist.

4. Abgabesystem nach Anspruch 1, wobei Zugausübung am ersten Ende der Halteschnur den lösbaren Knoten um das proximale Befestigungsteil der Emboliespirale festzieht.

5. Abgabesystem nach Anspruch 4, wobei Zugausübung am zweiten Ende der Halteschnur den lösbaren Knoten vom proximalen Befestigungsteil der Emboliespirale löst.

6. Abgabesystem nach Anspruch 1, wobei sich der lösbare Knoten nicht außerhalb des distalen Endes der Abgaberöhre erstreckt, wenn der lösbare Knoten am proximalen Befestigungsteil der Emboliespirale befestigt ist.

7. Abgabesystem nach Anspruch 1, wobei das Befestigungsteil eine Öse aufweist, die eine Öffnung bildet, der lösbare Knoten die Öffnung der Öse durchläuft und mindestens ein Abschnitt der Öse gekrümmt ist.

## Revendications

1. Système de mise en place de spirale d'embolisation, comprenant :
un microcathéter (10) qui inclut une extrémité proximale (14) définissant une ouverture proximale (15), une extrémité distale (16) définissant une ouverture distale (17) et une lumière (12) s'étendant entre elles ;
un tube de mise en place (20) disposé à coulissement dans la lumière du microcathéter, où le tube de mise en place inclut une extrémité proximale définissant une ouverture proximale, une extrémité distale (26) définissant une ouverture distale (27) et une lumière s'étendant entre elles ;
une spirale d'embolisation (30) qui inclut un élément d'attache proximal (32) configuré pour s'engager de manière réversible avec l'extrémité distale du tube de mise en place, et
un cordon comprenant une première extrémité (42a) et une seconde extrémité (44a), où le cordon forme une boucle suivant une longueur de la lumière du tube de mise en place, et où une partie (42b, 44b) du cordon forme un nœud pouvant être desserré (46) autour de l'élément d'attache proximal de la spirale d'embolisation, où une partie de l'élément d'attache proximal s'étend dans l'ouverture distale du tube de mise en place quand l'élément d'attache proximal est fixé à l'extrémité distale du tube de mise en place, ledit système de mise en place de spirale d'embolisation étant **caractérisé en ce que** l'élément d'attache proximal est dimensionné pour s'ajuster seulement partiellement dans l'ouverture distale du tube de mise en place.

2. Système de mise en place selon la revendication 1, où les première et seconde extrémités du cordon s'étendent à l'extérieur de l'extrémité proximale du microcathéter.

3. Système de mise en place selon la revendication 1, où le nœud pouvant être desserré est un nœud coulant ou un nœud de brigand.

4. Système de mise en place selon la revendication 1, où l'application d'une tension à la première extrémité du cordon resserre le nœud pouvant être desserré autour de l'élément d'attache proximal de la spirale d'embolisation.

5. Système de mise en place selon la revendication 4, où l'application d'une tension à la seconde extrémité du cordon desserre le nœud pouvant être desserré de l'élément d'attache proximal de la spirale d'embolisation.

6. Système de mise en place selon la revendication 1, où le nœud pouvant être desserré ne s'étend pas à l'extérieur de l'extrémité distale du tube de mise en place quand le nœud pouvant être desserré est attaché à l'élément d'attache proximal de la spirale d'embolisation.

7. Système de mise en place selon la revendication 1, où l'élément d'attache comprend un œillet définissant une ouverture, le nœud pouvant être desserré passe par l'ouverture de l'œillet, et au moins une partie de l'œillet est incurvée.
